(19) Europäisches Patentamt
European Patent Office
Office européen des brevets

(11) EP 2 772 536 B1

(12) EUROPEAN PATENT SPECIFICATION

(45) Date of publication and mention
of the grant of the patent:
**03.05.2017 Bulletin 2017/18**

(21) Application number: **12842918.0**

(22) Date of filing: **25.10.2012**

(51) Int Cl.:
*C12N 9/00* (2006.01)    *C12N 9/02* (2006.01)
*C12N 9/14* (2006.01)    *C12N 9/96* (2006.01)

(86) International application number:
**PCT/JP2012/077627**

(87) International publication number:
**WO 2013/062054 (02.05.2013 Gazette 2013/18)**

(54) **COMPOSITION AND METHOD FOR PRODUCING SAME**

ZUSAMMENSETZUNG UND HERSTELLUNGSVERFAHREN DAFÜR

COMPOSITION ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2011 JP 2011238013**

(43) Date of publication of application:
**03.09.2014 Bulletin 2014/36**

(73) Proprietors:
• **Sunstar Engineering Inc.**
**Osaka 569-1134 (JP)**
• **Sunstar Singapore Pte. Ltd.**
**Singapore Science ParkII 117684 (SG)**

(72) Inventors:
• **MIYAO, Haruka**
**Takatsuki-shi**
**Osaka 569-1134 (JP)**
• **AJIMA, Yuzuru**
**Takatsuki-shi**
**Osaka 569-1134 (JP)**
• **OKA, Toru**
**Takatsuki-shi**
**Osaka 569-1134 (JP)**

• **LIAUW, Denny**
**Singapore 117684 (SG)**

(74) Representative: **Wilkinson, Stephen John**
**Stevens, Hewlett & Perkins**
**1 St. Augustine's Place**
**Bristol BS1 4UD (GB)**

(56) References cited:
**WO-A1-2008/081948      JP-A- 2003 071 472
JP-A- 2007 105 677      JP-A- 2008 049 243
JP-A- 2009 225 748**

• **Joachim Dzubiella: "Explicit and implicit
modeling of nanobubbles in hydrophobic
confinement", Annals of the Brazilian Academy
of Sciences, 1 March 2010 (2010-03-01), pages
3-12, XP055189879, Rio de Janeiro Retrieved from
the Internet:
URL:http://www.scielo.br/pdf/aabc/v82n1/a0
2v82n1.pdf [retrieved on 2015-05-18]**
• **SHUICHI YAMAMOTO: 'Enzyme inactivation in
drying : relation between enzyme heat stability
and water content' NEW FOOD INDUSTRY vol. 36,
no. 8, 1994, pages 71 - 87, XP008172661**

## Description

### Technical Field

The present invention relates to a composition comprising an enzyme and water containing ultrafine gas bubbles having a mode particle size of no greater than 500 nm (such bubbles are also referred to as nanobubbles or NBs), as well as a method for stabilizing an enzyme composition which comprises mixing an enzyme with water containing ultrafine gas bubbles having a mode particle size of no greater than 500 nm and a concentration of at least $10^6$ bubbles per ml.

### Background Art

Proteins such as enzymes, antibodies and peptides are extensively used in detergents, industry, cosmetics, food processing, pharmaceuticals, diagnosis/examination, and biosensors. Water-soluble preparations/forms of proteins are easier to handle than powders and, because of this and other advantages, are commonly used in fields that involve the use of enzymes in large quantities. On the other hand, however, enzymes in aqueous solution are generally considerably instable and low in keeping quality as compared with the case where they are in powder form and, hence, are incapable of maintaining their physiological activity for an extended period of time. A conventionally known method for supplying proteins without lowering their physiological activity was to purify and stabilize the protein by freeze-drying or other means to avoid heat application and then supply the resulting preparation. Known methods for stabilizing proteins in aqueous solution include a process involving a step of incorporating polyhydric alcohols such as glycerin as stabilizers for uricase or peroxidase (Patent Document 1: JP Hei 6-70798A), a technique involving a step of adding bovine serum albumin or saccharides such as glucose or amino acids such as lysine to an aqueous solution containing cholesterol oxidase (Patent Document 2: JP Hei 8-187095A), and a technique in which an organic compound such as guanidine hydrochloride, urea or pyridine is added as a stabilizer to any kind of proteins in aqueous solution (Patent Document 3: JP 2011-67202A).

These known techniques, however, have had several problems. Problems with the freeze-drying based method, for example, are that it is not applicable to proteins that will denature upon dehydration and that deterioration tends to result from moisture absorption and oxidation during the drying step, presenting the need to re-condition the aqueous solution of enzyme, which is simply a cumbersome procedure to adopt. The techniques of stabilizing proteins in aqueous solution are one of using polyhydric alcohols to stabilize uricase or peroxidase and another of using bovine serum albumin, saccharides or amino acids to stabilize an aqueous solution of cholesterol oxidase; but intending to stabilize only particular kinds of proteins, each of these methods has had the disadvantage of being limited in applicability. The method for using organic compounds such as guanidine hydrochloride to stabilize aqueous solutions of proteins has wide applicability but, on the other hand, it has the disadvantage of being inapplicable if the enzyme preparation formulated as a mixture containing a certain substance that reacts with the organic compound; as another problem, the concentration of the organic compound to be added must be adjusted to an appropriate value but again this is a cumbersome procedure to adopt.

### Prior Art Literature

### Patent Literature

Patent Document 1: JP Hei 6-70798A
Patent Document 2: JP Hei 8-187095A
Patent Document 3: JP 2011-67202A

Bubbles and nanobubbles have been used in various technologies. Compositions comprising nanobubbles in an aqueous solution and methods of stabilising proteins using such compositions are described in "New Food Industry", 1994 vol.36, No.8, pp 71-87. JP 2009 225748A discloses a method of rearing animals comprising giving the animals activated water in which activity is increased by applying ultrasound and complex sound waves. It is indicated that the activated water is one in which nanobubbles are created. In JP 2003 071472A a liquid treating system is disclosed as comprising a mineral solution, vitamins and an enzyme. This system is produced by a method which comprises mixing air into the system to create superfine air bubbles. WO 2008/081948A relates to a method for manufacturing soymilk which includes a step for performing submerged grinding using a grinder on soybeans along with a grinding solution, a preheating step for preheating a soybean paste obtained in the submerged grinding step, and a heating step for further heating the soybean paste obtained in the preheating step. Nanobubble water may be added by injection in the heating step. A method of separating a hydrophobic compound generated by a biological reaction is described in JP 2008 049243A. According to this method, the hydrophobic compound in an aqueous medium is contacted with and captured by air bubbles, such as nanobubbles, in the medium. JP 2007-105677A discloses using activated water in an enzyme reaction. The activated water is obtained by applying complex sound waves and ultrasonic waves to the water. The water may contain nanobubbles. Joachim Dzubiella describes, in "Explicit and implicit modeling of nanobubbles in hydrophobic confinement", Annals of the Brazilian Academy of Sciences, 1 March 2010 (2010-03-01), pages 3-12, the formation of nanobubbles between hydrophobic surfaces and mentions that nanobubbles are thought to play a crucial role in protein stability.

## Summary of Invention

### Problem to be Solved by the Invention

An object of the present invention is to provide novel, stable enzyme compositions that are free from the above-described problems. Another object of the present invention is to provide methods for stabilizing enzymes without involving the above-described problems.

### Means for Solving the Problems

With a view to solving the above-described problems, the present inventors conducted intensive studies and found that stable enzyme compositions were obtained by mixing enzymes with water containing ultrafine gas bubbles; the present invention has been accomplished on the basis of this finding.

The present invention provides a composition comprising an enzyme and water containing ultrafine gas bubbles having a mode particle size of no greater than 500 nm wherein the mode particle concentration of ultrafine gas bubbles is at least $1 \times 10^6$ bubbles per millilitre. According to an embodiment, the particle concentration of bubbles having a particle diameter of no greater than 1000 nm is at least $5 \times 10^7$ bubbles per millilitre.

The present invention also provides a method for stabilizing an enzyme composition which comprises mixing an enzyme with water containing ultrafine gas bubbles having a mode particle size of no greater than 500 nm and wherein the mode particle concentration of ultrafine gas bubbles is at least $1 \times 10^6$ bubbles per millilitre. According to an embodiment, the particle concentration of bubbles having a particle diameter of no greater than 1000 nm is at least $5 \times 10^7$ bubbles per millilitre.

In the present invention, the interior of the above-described ultrafine bubbles may be filled with one or more gases selected from a wide range of gases which include, but are not limited to, air, oxygen, hydrogen, nitrogen, carbon dioxide, argon, neon, xenon, fluorinated gases, and inert gases.

The enzymes that can be used in the enzyme compositions of the present invention are not particularly limited and the following may be given as examples that can be used.

Enzymes that can be used are oxidoreductases (e.g. cholesterol oxidase, glucose oxidase, ascorbate oxidase, polyphenol oxidase, and peroxidase); transferases (e.g. acyltransferase, sulfotransferase, and transglucosidase); hydrolases (e.g. protease, serine protease, amylase, lipase, cellulose, glucoamylase, and lysozyme); lyases (e.g. pectin lyase); isomerases (e.g. glucose isomerase); synthases (e.g. fatty acid synthase, phosphate synthase, citrate synthase, hyaluronate synthase, and carbonate dehydratase).

In a preferred mode of the present invention, the enzyme is a water-soluble enzyme; most preferably, the enzyme may be at least one enzyme selected from peroxidase, protease, cellulose, amylase, and lipase.

The amount of enzymes to be used varies with factors such as their type and usage. Preferred amounts can be determined as appropriate by experiment and enzymes can generally be used in the range from 1 ng/ml to 300 mg/ml, preferably from 10 ng/ml to 100 mg/ml, and more preferably from 30 ng/ml to 50 mg/ml.

The water to be used in the present invention may be selected from tap water, purified water, ion-exchanged water, pure water, ultrapure water, deionized water, distilled water, buffered water, clean water, natural water, filtered water, highly pure water, potable water, and electrolyzed water, but these are not the sole examples of water that can be used in the present invention.

If desired, water-soluble solvents may be added and examples include alcohols, glycols, glycerins, ethers, ketones, and esters.

### Advantageous Effects of the Invention

The enzyme-containing compositions of the present invention are highly stable and, in particular, they exhibit high pH stability (being stable in the face of pH changes), high temperature stability (reducing the temperature effect), and high photostability (reducing the effect of light).

## Brief Description of the Drawings

FIG.1 is a graph showing the result of measuring the particle size distribution of bubbles within water containing ultrafine bubbles.

FIG.2 is a graph showing the result of measuring the particle size distribution of bubbles within purified water as specified in the Japanese Pharmacopoeia.

FIG.3 is a graph showing the results of a test for measurement of catalase stability.

FIG.4 is a graph showing the results of a test for measurement of lipase stability.

## Modes for Carrying Out the Invention

The ultrafine bubbles to be used in the present invention have a mode particle size of no greater than 500 nm, preferably no greater than 300 nm, more preferably no greater than 150 nm, and most preferably no greater than 100 nm, and the concentration of the bubbles with the mode particle size is preferably at least $1 \times 10^6$, more preferably at least $3 \times 10^6$, even more preferably at least $5 \times 10^6$, still more preferably at least $7 \times 10^6$, yet more preferably at least $1 \times 10^7$, still more preferably at least $5 \times 10^7$, even more preferably at least $9 \times 10^7$, yet more preferably at least $1 \times 10^8$, even more preferably at least $5 \times 10^8$, and most preferably at least $9 \times 10^8$ bubbles per millilitre.

In the present invention, the total particle concentration is preferably at least $5 \times 10^7$, more preferably at least $7 \times 10^7$, even more preferably at least $8 \times 10^7$, still more preferably at least $1 \times 10^8$, yet more preferably at least $6 \times 10^8$, still more preferably at least $1 \times 10^9$, even more preferably at least $3 \times 10^9$, yet more preferably at least $5 \times 10^9$, even more preferably at least $7 \times 10^9$, still more preferably at least $1 \times 10^{10}$, yet more preferably at least $2 \times 10^{10}$, even more preferably at least $5 \times 10^{10}$, and most preferably at least $7 \times 10^{10}$ bubbles per millilitre. In a further preferred mode, bubbles larger than 1000 nm are virtually absent.

The particle diameter of the ultrafine bubbles to be used in the present invention is so small that it cannot be measured correctly with an ordinary particle size distribution analyzer. Hence, hereinafter, numerical values are employed that were obtained by measurements with the nanoparticle size analyzing system NanoSight Series (product of NanoSight Ltd.). The nanoparticle size analyzing system NanoSight Series (product of NanoSight Ltd.) measures the velocity of nanoparticles moving under Brownian motion and calculates the diameters of the particles from the measured velocity. A mode particle size can be verified from the size distribution of the particles present and represents the particle diameter for the case where the particles present at a maximum number.

It should be noted here that the particle diameter and number of ultrafine bubbles as referred to in the present invention are represented by numerical values as measured at the point in time when 24 hours have passed after the formulation of the composition.

It should also be noted that the composition of the present invention may contain additives including, but not limited to, antiseptics and stabilizers. Antiseptics that can be used include, but are not limited to, polyhexamethylene biguanide and paraben. Stabilizers that can be used include, but are not limited to, saccharides, antibiotics, aminoglycosides, organic acids, coenzymes, and amino acids. The composition of the present invention may also contain surfactants. Surfactants may be added as appropriate for use and other conditions, not only in the case where substances either insoluble or slightly soluble in water are contained as additives but also in the case of using water-soluble additives.

The zeta potential at surfaces of ultrafine bubbles have a certain effect on the stability of the bubbles. The surfaces of the ultrafine bubbles used in the present invention are electrically charged to provide a zeta potential of at least 5 mV, preferably at least 7 mV, more preferably at least 10 mV, even more preferably at least 20 mV, still more preferably at least 25 mV, and most preferably at least 30 mV, in absolute value. The absolute value of zeta potential is in proportion to the viscosity coefficient of a solution as divided by the dielectric constant of the solution, so the lower the temperature condition under which the water containing the ultrafine bubbles is mixed with an enzyme, the more likely it is that the bubbles have higher stability.

The ultrafine bubbles to be used in the present invention can be generated by any known means, such as the use of a static mixer, the use of a venturi tube, cavitation, vapor condensation, sonication, swirl formation, dissolution under pressure, or fine pore formation. A preferred method of bubble generation is by forming a gas-liquid mixture and shearing it.

An advantageous apparatus for generating ultrafine bubbles by the gas-liquid mix and shear method is disclosed in Japanese Patent No. 4118939. In this apparatus, the greater part of a gas-liquid mixture in fluid form introduced into a fluid swirling compartment does not simply flow toward the discharge port as in the apparatus already described in the Prior Art section but it first flows forming a swirl in the direction away from the discharge port. The swirl reaching the first end-wall member turns around and flows back toward the second end-wall member; since the returning swirl has a smaller radius of rotation than the swirl flowing toward the first end-wall member, it flows at a higher velocity, creating a sufficient shear force on the gas within the liquid to promote the formation of finer bubbles.

An aqueous solution of an enzyme is treated with a suitable apparatus to generate ultrafine bubbles in it, whereby the composition of the present invention can be produced that has the enzyme dissolved in the water. The composition of the present invention can also be produced by dissolving an enzyme in the water containing ultrafine bubbles. The water containing ultrafine bubbles may have the above-defined mode particle size and number of bubbles.

The foregoing description of the present invention and the description of the Examples that follow are only intended to provide a detailed explanation of various exemplary embodiments of the present invention and skilled artisans can make various improvements and changes of the embodiments disclosed herein without departing from the scope of the present invention. Therefore, the description herein will in no way limit the scope of the present invention, which shall be determined only by the recitation in the appended claims.

**Examples**

Preparation of Water Containing Atmospheric Ultrafine Bubbles

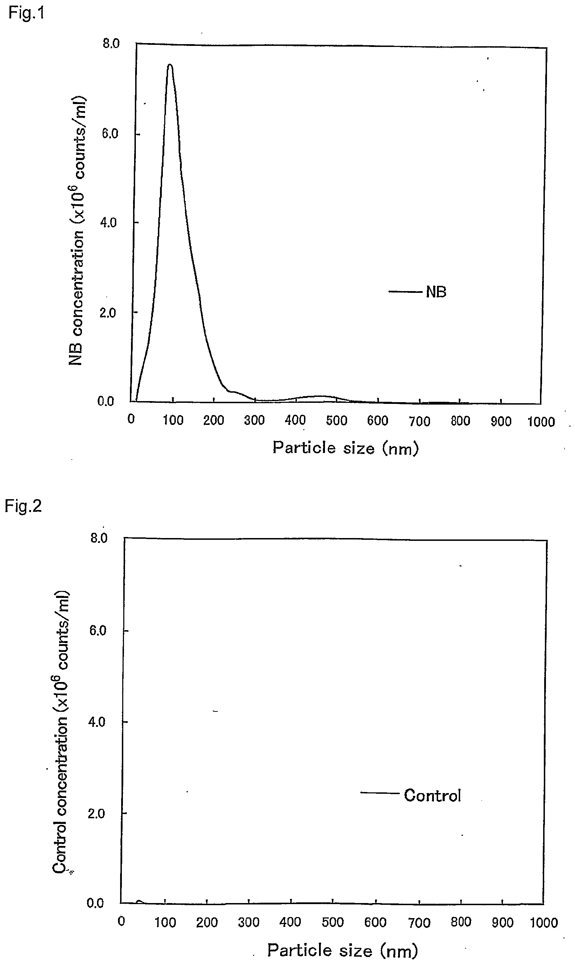

Ultrafine bubbles were generated in purified water (Japanese Pharmacopoeia) using BAVITAS of KYOWA KISETSU which was a device for generating ultrafine bubbles by the gas-liquid mix and shear method. The particle diameters of the generated ultrafine bubbles were measured with the nanoparticle size analyzing system NanoSight Series (product of NanoSight Ltd.) The result is shown in FIG. 1. The horizontal axis of the graph represents the particle diameter in nanometers and the vertical axis represents the number of NB particles (the number of nanobubble particles) per millimeter ($10^6$/ml). FIG. 2 shows the result of a measurement of fine bubbles in the purified water of the Japanese Pharmacopoeia.

The water containing the generated ultrafine bubbles had a mode particle size of 86 nm; the particle concentration at the mode particle size was $7.57 \times 10^6$ bubbles per milliliter and the total particle concentration was $6.86 \times 10^8$ bubbles per milliliter.

The purified water of the Japanese Pharmacopoeia had such low particle concentrations that the size distribution was not normal distribution ; the result of the measurement was therefore attributed to noise.

In the following Examples, water containing atmospheric ultrafine bubbles was prepared by the same method as described above, and the blanks as Comparative Examples used the purified water of the Japanese Pharmacopoeia in place of the water containing atmospheric ultrafine bubbles.

1. Stability of peroxidase at 20 °C or 50 °C for 10 days

To water containing atmospheric ultrafine bubbles, streptavidin peroxidase was added at a concentration of 50 ng/ml and the resulting mixture was dispensed into tubes in 1.0-ml portions; the tubes were then closed with an airtight stopper and stored at 20 °C or 50 °C for 1, 3 or 10 days. Subsequently, the stored aqueous solution of streptavidin peroxide was allowed to develop color by mixing 100 μl of the solution with 100 μl of a substrate solution prepared as described below.

(Method for preparing the substrate solution)

The reagents according to the recipe indicated below were mixed to prepare the substrate solution.

o-Phenylenediamine tablet (product of SIGMA-ALDRICH): one
70 mM citrate buffer (citric acid/sodium phosphate; pH 5.0): 12.5 ml
Hydrogen peroxide solution: 5 μl

(Methods for calculating the concentration of enzyme and the percent residual enzyme activity)

To the sample, 50 μl of a 4 N $H_2SO_4$ solution was added to quench the color reaction and thereafter the absorbance at 492 nm ($A_{492}$) was measured with a micro-plate reader (product of Thermo Fisher Scientific Inc.) The concentration of the enzyme was determined from a calibration curve constructed by using streptavidin peroxidase. The percent residual enzyme activity was calculated from the following equation, where A is the initial enzyme activity of the sample and B is the enzyme activity after storage:

$$\text{Residual enzyme activity (\%)} = (1 - (A-B)/A) \times 100$$

Note that the concentration at the mode particle size was calculated as a measured concentration value at the mode particle size ($\times 10^6$ bubbles/ml) multiplied by the dilution ratio at measurement. The total particle concentration was calculated as a measured concentration value at the total particle concentration ($\times 10^8$ bubbles/ml) multiplied by the dilution ratio at measurement.

The results are shown in the following Tables 1 and 2.

Table 1

| | | Comp. Ex. 1 | Comp. Ex. 2 | Comp. Ex. 2 | Ex.1 | Ex. 2 | Ex. 3 |
|---|---|---|---|---|---|---|---|
| Test conditions | Temperature of heat treatment (°C) | 20 | 20 | 20 | 20 | 20 | 20 |
| | Time of heat treatment (days) | 1 | 3 | 10 | 1 | 3 | 10 |
| | Mode particle size (nm) | - | - | - | 86 | 86 | 86 |
| | Concentration at mode particle size ($\times 10^6$ bubbles/ml) | - | - | - | 7.57 | 7.57 | 7.57 |
| | Total particle concentration ($\times 10^8$ bubbles/ml) | - | - | - | 6.86 | 6.86 | 6.86 |
| Enzyme activity | Residual enzyme (%) | 100.0 | 104.7 | 77.9 | 112.0 | 123.6 | 98.1 |

Table 2

| | | Comp. Ex. 4 | Comp. Ex. 5 | Comp. Ex. 6 | Ex. 4 | Ex. 5 | Ex. 6 |
|---|---|---|---|---|---|---|---|
| Test conditions | Temperature of heat treatment (°C) | 50 | 50 | 50 | 50 | 50 | 50 |
| | Time of heat treatment (days) | 1 | 3 | 10 | 1 | 3 | 10 |
| | Mode particle size (nm) | - | - | - | 86 | 86 | 86 |
| | Concentration at mode particle size ($\times 10^6$ bubbles/ml) | - | - | - | 7.57 | 7.57 | 7.57 |
| | Total particle concentration ($\times 10^8$ bubbles/ml) | - | - | - | 6.86 | 6.86 | 6.86 |
| Enzyme activity | Residual enzyme (%) | 52.4 | 34.3 | 11.3 | 108.7 | 104.5 | 75.1 |

Examples 1 to 3 show the results in the case of storage at 20 °C for 1, 3, and 10 days. Examples 4 to 6 show the results in the case of storage at 50 °C for 1, 3, and 10 days. The comparison at 20 °C reveals marked improvements in percent residual enzyme. The results at 50 °C were also superb.

2. Stability of enzyme to heat (80 °C)

(Preparation of enzyme solutions)

To water containing atmospheric ultrafine bubbles, streptavidin peroxidase was added at a concentration of 50 ng/ml and the resulting mixture was dispensed into tubes in 1.0-ml portions; the tubes were then closed with an airtight stopper and stored under the condition of 80 °C for 30 minutes. In the process, the mode particle concentration of the water containing atmospheric ultrafine bubbles was adjusted to an order of $10^6$ counts (Examples 7 and 8), an order of $10^7$ counts (Examples 9 and 10), and an order of $10^8$ counts (Examples 11 and 12), per milliliter.

Subsequently, the stored aqueous solution of streptavidin peroxide was allowed to develop color by mixing 100 μl of the solution with 100 μl of a substrate solution prepared from the reagents set out below.

(Preparation of the substrate solution)

The reagents according to the recipe indicated below were mixed to prepare the substrate solution.

o-Phenylenediamine tablet (product of SIGMA-ALDRICH): one
70 mM citrate buffer (citric acid/sodium phosphate; pH 5.0): 12.5 ml
Hydrogen peroxide solution: 5 μl

(Methods for calculating the concentration of enzyme and the percent residual enzyme activity)

To the sample, 50 μl of a 4 N $H_2SO_4$ solution was added to quench the color reaction and thereafter the absorbance at 492 nm was measured with a micro-plate reader (product of Thermo Fisher Scientific Inc.) The concentration of the enzyme was determined from a calibration curve constructed by using streptavidin peroxidase. The percent residual enzyme activity was calculated from the following equation, where A is the initial enzyme activity of the sample and B is the enzyme activity after storage:

$$\text{Residual enzyme activity (\%)} = (1 - (A-B)/A) \times 100$$

The results are shown in the following Table 3.

Obviously, the increased total particle concentration provided better results.

It is interesting to note that the residual enzyme was 0.0% in Example 8 and 15.2% in Example 10. Although the result of Example 10 was better than that of Comparative Example 8, the condition of 80 °C x 80 min is considered to require that the concentration at the mode particle size and the total particle concentration be desirably on the orders of $10^8$ and $10^{10}$, respectively, in terms of bubble counts as in Example 12.

Table 3

| | | Comp. Ex. 7 | Comp. Ex. 8 | Ex. 7 | Ex. 8 | Ex. 9 (calculated particle concentration) | Ex. 10 (calculatd particle concentration) | Ex. 11 (calculated particle concentration) | Ex. 12 (calculated particle concentration) |
|---|---|---|---|---|---|---|---|---|---|
| Test conditions | Temperature of heat treatment (°C) | 80 | 80 | 80 | 80 | 80 | 80 | 80 | 80 |
| | Time of heat treatment (min) | 30 | 80 | 30 | 80 | 30 | 80 | 30 | 80 |
| | Mode particle size (nm) | - | - | 79 | 79 | 76 | 76 | 76 | 76 |
| | Concentration at mode particle size ($\times 10^6$ bubbles/ml) | - | - | 1.13 | 1.13 | 91.5 | 91.5 | 915 | 915 |
| | Total particle concentration ($\times 10^8$ bubbles/ml) | - | - | 0.89 | 0.89 | 70.8 | 70.8 | 708 | 708 |
| Enzyme activity | Residual enzyme (%) | 0.73 | 0.0 | 24.7 | 0.0 | 76.2 | 15.2 | 95.5 | 57.6 |

3. Stability of enzyme to pH

Using water containing atmospheric ultrafine bubbles, a citrate buffer (pH 4.6), an acetate buffer (pH 5.7), a phosphate buffer (pH 7.0), and a borate buffer (pH 8.9) were prepared; to each of these buffers, streptavidin peroxidase was added at a concentration of 50 ng/ml. The resulting mixtures were each dispensed into tubes in 1.0-ml portions; the tubes were then closed with an airtight stopper and stored under the condition of 80 °C for 20 minutes. Subsequently, the stored aqueous solution of streptavidin peroxide was allowed to develop color by mixing 100 $\mu$l of the solution with 100 $\mu$l of a substrate solution prepared from the reagents set out below.

(Preparation of the substrate solution)

The reagents according to the recipe indicated below were mixed to prepare the substrate solution.

o-Phenylenediamine tablet (product of SIGMA-ALDRICH): one

70 mM citrate buffer (citric acid/sodium phosphate; pH 5.0): 12.5 ml

Hydrogen peroxide solution: 5 $\mu$l

(Methods for calculating the concentration of enzyme and the percent residual enzyme activity)

To the sample, 50 $\mu$l of a 4 N $H_2SO_4$ solution was added to quench the color reaction and thereafter the absorbance at 492 nm was measured with a micro-plate reader (product of Thermo Fisher Scientific Inc.) The concentration of the enzyme was determined from a calibration curve constructed by using streptavidin peroxidase. The percent residual enzyme activity was calculated from the following equation, where A is the initial enzyme activity of the sample and B is the enzyme activity after storage:

$$\text{Residual enzyme activity (\%)} = (1 - (A-B)/A) \times 100$$

The results are shown in the following Table 4.
Obviously, the present invention provided excellent stability over a wider pH range.

Table 4

| | | Comp. Ex. 9 | Comp. Ex. 10 | Comp. Ex. 11 | Comp. Ex. 12 | Ex. 13 | Ex 14 | Ex. 15 | Ex. 16 |
|---|---|---|---|---|---|---|---|---|---|
| Test conditions | Temperature of heat treatment (°C) | 80 | 80 | 80 | 80 | 80 | 80 | 80 | 80 |
| | Time of heat treatment (min) | 20 | 20 | 20 | 20 | 20 | 20 | 20 | 20 |
| | Buffer | Citrate | Acetate | Phosphate | Borate | Citrate | Acetate | Phosphate | Borate |
| | pH | 4.6 | 5.7 | 7.0 | 8.9 | 4.6 | 5.7 | 7.0 | 8.9 |
| | Mode particle size (nm) | - | - | - | - | 86 | 86 | 86 | 86 |
| | Concentration at mode particle size ($\times 10^6$ bubbles/ml) | - | - | - | - | 7,57 | 7.57 | 7.57 | 7.57 |
| | Total particle concentration ($\times 10^8$ bubbles/ml) | - | - | - | - | 6.86 | 6.86 | 6.86 | 6.86 |
| Enzyme activity | Residual enzyme (%) | 24.3 | 65.0 | 62.4 | 42.6 | 75.5 | 83.0 | 95.3 | 72.6 |

4. Heat (80 °C) stability of protease

To water containing atmospheric ultrafine bubbles, protease was added at a concentration of 10 mg/ml and after standing at 80 °C for 30 minutes, the resulting mixture was dispensed into tubes in 990-$\mu$l portions. After adding a solution of Azocasein Tris-Cl (1.3 M) and $CaCl_2$ (20 mM) in an amount of 10 $\mu$l to give a concentration of 0.05% (w/v), the tubes were placed on a heating block set at 60 °C (product of YAMATO SCIENTIFIC CO., LTD), closed with an airtight stopper, and then stored for 30 minutes.

Subsequently, 1.1 ml of 10% (w/v) trichloroacetic acid was added and the mixture was left to stand at room temperature (25-27 °C) for 30 minutes to quench the reaction. To separate the precipitating protein, the mixture was centrifuged at 13000 x g for 10 minutes and the supernatant (1 ml) was allowed to develop color in a separate tube filled with 1 ml of 1 M NaOH.

(Methods for calculating the enzyme activity and the percent residual enzyme activity)

The prepared enzyme solution was subjected to a measurement of absorbance at 450 nm ($A_{450}$) with a microplate reader (product of Thermo Fisher Scientific Inc.) A blank was subjected to the same measurement of absorbance at 450 nm ($A_{450b}$). Using the two measurement values, enzyme activity was calculated from the following formula.

$$\text{Enzyme activity (U/ml)} = (A_{450} - A_{450b})/(0.001 \times 30)$$

\* One unit (U) of protease activity is defined as "an increase of 0.001 per minute in the absorbance at 450 nm."

In the next step, the percent residual enzyme activity was calculated from the following equation, where A is the initial enzyme activity of the sample and B is the enzyme activity after 30-min storage:

$$\text{Residual enzyme activity (\%)} = (1 - (A-B)/A) \times 100$$

The results are shown in the following Table 5.
Obviously, the present invention also provided outstanding heat stability for protease.

Table 5

| | | Comp. Ex. 13 | Ex. 17 (calculated particle concentration) |
|---|---|---|---|
| Test conditions | Temperature of heat treatment (°C) | 80 | 80 |
| | Time of heat treatment (min) | 30 | 30 |
| | Mode particle size (nm) | - | 99 |
| | Concentration at mode particle size (x$10^6$ bubbles/mi) | - | 303 |
| | Total particle concentration (x$10^8$ bubbles/ml) | - | 215 |
| Enzyme activity | Residual enzyme (%) | 24.8 | 65.0 |

5. Stability of catalase

(Testing procedure)

(1) Catalase was dissolved in either purified water or water containing ultrafine bubbles (mode particle size, 100 nm; mode particle concentration, 8.77 x $10^6$/mL; total particle concentration at a particle diameter of no greater than 1000 nm, 6.18 x $10^8$/mL) to prepare an aqueous solution having a catalase concentration of 1 mg/mL (3809 Units/mL.)
(2) The aqueous catalase solution (1 mL) dispensed in microtubes was incubated in a thermo-shaker at 500 rpm and 62.5 °C for a predetermined period of time (20, 30, 40 or 50 min.) Thereafter, the aqueous solution was subjected to a treatment with a centrifuge (14000 rpm x 5 min) to cause precipitation.

(3) A 100-$\mu$L portion of the supernatant of the thus treated aqueous catalase solution, 200 $\mu$L of 3% aqueous hydrogen peroxide, 200 $\mu$L of a phosphate buffer (500 mM, pH 5.8), and 100 $\mu$L of purified water were metered into tubes and subjected to reaction at room temperature for 15 minutes.

(4) After the reaction, heat treatment (100 °C x 5 min) was conducted to achieve complete deactivation of the enzyme.

(5) The reaction mixture was metered in an amount of 100 $\mu$L and the absorbance at 290 nm was measured. From the measured values of absorbance, the percent decomposition of hydrogen peroxide was calculated using a calibration curve.

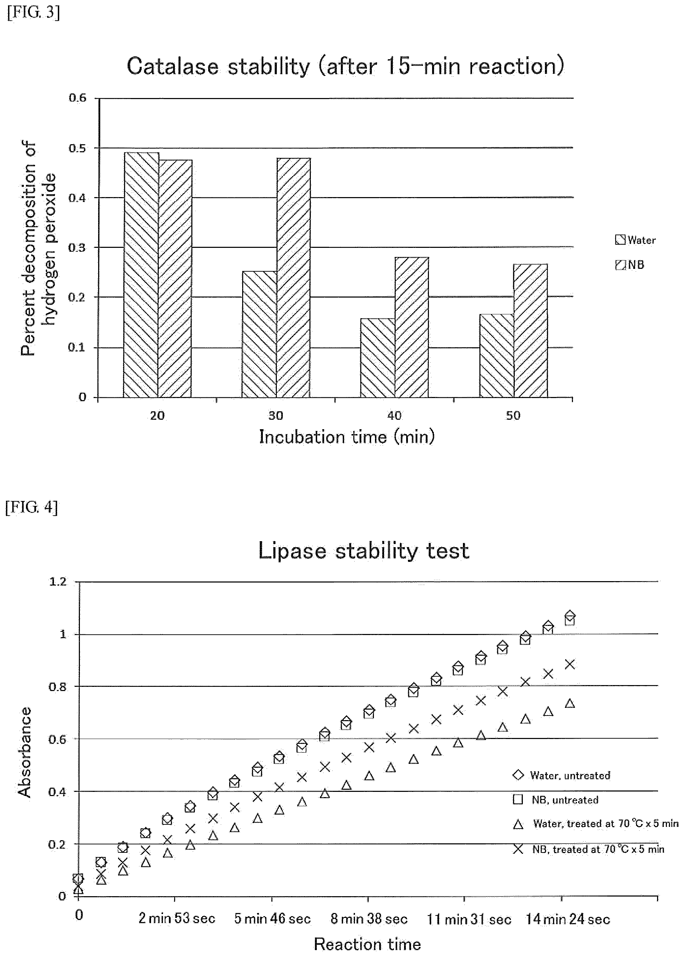

The results are shown in Fig. 3.

6. Stability of lipase

(Testing procedure)

(1) Lipase was dissolved in either purified water or water containing ultrafine bubbles (mode particle size, 113 nm; mode particle concentration, 36.4 x $10^6$/mL; total particle concentration at a particle diameter of greater than 1000 nm, 20.9 x $10^8$/mL) to prepare an aqueous solution having a lipase concentration of 0.02 mg/mL (23.52 Units/mL.)

(2) The thus prepared aqueous lipase solution was incubated at 70 °C for 5 minutes.

(3) A 50-$\mu$L portion of the thus treated aqueous lipase solution, 100 $\mu$L of a substrate solution prepared by the method just described below, and 50 $\mu$L of a Tris-HCl buffer (pH 8.2) were subjected to reaction at room temperature for predetermined periods of time.

The reaction mixture was metered in a predetermined amount and the absorbance at 410 nm was measured.

The results are shown in Fig. 4.

(Preparation of the substrate solution)

Purified water (100 mL) was mixed with 0.0135 g of p-nitrophenyl laurate, 0.017 g of sodium dodecyl sulfate, and 1.0 g of Triton X-100 and the added ingredients were dissolved in the water by heating the mixture at 65 °C; the solution was subsequently cooled.

## Claims

1. A composition comprising an enzyme and water containing ultrafine gas bubbles having a mode particle size of no greater than 500 nm, wherein the mode particle concentration of ultrafine gas bubbles is at least 1 x $10^6$ bubbles per millilitre.

2. The composition according to claim 1, wherein the particle concentration of bubbles having a particle diameter of no greater than 1000 nm is at least 5 x $10^7$ bubbles per millilitre.

3. The composition according to either claim 1 or claim 2, wherein the ultrafine bubbles are filled with one or more gases selected from air, oxygen, hydrogen, nitrogen, carbon dioxide, argon, neon, xenon, fluorinated gases, and inert gases.

4. The composition according to either claim 1 or claim 2, wherein the enzyme is peroxidase, protease, cellulose, amylase, or lipase.

5. A method for stabilizing an enzyme composition which comprises mixing an enzyme with water containing ultrafine gas bubbles having a mode particle size of no greater than 500 nm and wherein the mode particle concentration is at least 1 x $10^6$ bubbles per millilitre.

6. The method according to claim 5, wherein the water containing ultrafine bubbles is such that the particle concentration of bubbles having a particle diameter of no greater than 1000 nm is at least 5 x $10^7$ bubbles per millilitre.

**7.** The method according to either claim 5 or claim 6, wherein the ultrafine bubbles include one or more gases selected from air, oxygen, hydrogen, nitrogen, carbon dioxide, argon, neon, xenon, fluorinated gases, and inert gases.

**8.** The method according to claim 5 or claim 7, wherein the enzyme is peroxidase, protease, cellulose, amylase, or lipase.

**Patentansprüche**

**1.** Zusammensetzung, umfassend ein Enzym und Wasser, das ultrafeine Gasbläschen mit einer Gipfel-Teilchengröße von maximal 500 nm enthält, wobei die Gipfel-Teilchenkonzentration von ultrafeinen Gasbläschen mindestens $1 \times 10^6$ Bläschen pro Millimeter beträgt.

**2.** Zusammensetzung nach Anspruch 1, wobei die Teilchenkonzentration von Bläschen mit einem Teilchendurchmesser von maximal 1000 nm mindestens $5 \times 10^7$ Bläschen pro Milliliter beträgt.

**3.** Zusammensetzung nach Anspruch 1 oder 2, wobei die ultrafeinen Bläschen mit einem oder mehreren Gasen gefüllt sind, die aus Luft, Sauerstoff, Wasserstoff, Stickstoff, Kohlendioxid, Argon, Neon, Xenon, fluorierten Gasen und inerten Gasen ausgewählt sind.

**4.** Zusammensetzung nach Anspruch 1 oder 2, wobei das Enzym Peroxidase, Protease, Cellulose, Amylase oder Lipase ist.

**5.** Verfahren zur Stabilisierung einer Enzymzusammensetzung, das ein Mischen eines Enzyms mit Wasser umfasst, das ultrafeine Gasbläschen mit einer Gipfel-Teilchengröße von maximal 500 nm enthält, und wobei die Gipfel-Teilchenkonzentration mindestens $1 \times 10^6$ Bläschen pro Millimeter beträgt.

**6.** Verfahren nach Anspruch 5, wobei das Wasser, das die ultrafeinen Bläschen enthält, derart ist, dass die Teilchenkonzentration von Bläschen mit einem Teilchendurchmesser von maximal 1000 nm mindestens $5 \times 10^7$ Bläschen pro Milliliter beträgt.

**7.** Verfahren nach Anspruch 5 oder 6, wobei die ultrafeinen Bläschen ein oder mehrere Gase enthalten, die aus Luft, Sauerstoff, Wasserstoff, Stickstoff, Kohlendioxid, Argon, Neon, Xenon, fluorierten Gasen und inerten Gasen ausgewählt sind.

**8.** Verfahren nach Anspruch 5 oder 7, wobei das Enzym Peroxidase, Protease, Cellulose, Amylase oder Lipase ist.

**Revendications**

**1.** Composition comprenant une enzyme et de l'eau contenant des bulles de gaz ultrafines ayant une taille modale de particules inférieure ou égale à 500 nm, dans laquelle la concentration modale de particules de bulles de gaz ultrafines est d'au moins $1 \times 10^6$ bulles par millilitre.

**2.** Composition selon la revendication 1, dans laquelle la concentration de particules de bulles ayant un diamètre de particule inférieur ou égal à 1 000 nm est d'au moins $5 \times 10^7$ bulles par millilitre.

**3.** Composition selon l'une des revendications 1 et 2, dans laquelle les bulles ultrafines sont remplies d'un ou plusieurs gaz choisi(s) parmi l'air, l'oxygène, l'hydrogène, l'azote, le dioxyde de carbone, l'argon, le néon, le xénon, des gaz fluorés et des gaz inertes.

**4.** Composition selon l'une des revendications 1 et 2, dans laquelle l'enzyme est la peroxydase, la protéase, la cellulose, l'amylase ou la lipase.

**5.** Procédé pour stabiliser une composition enzymatique, consistant à mélanger une enzyme avec de l'eau contenant des bulles de gaz ultrafines ayant une taille modale de particules inférieure ou égale à 500 nm, et dans laquelle la concentration modale des particules est d'au moins $1 \times 10^6$ bulles par millilitre.

**6.** Procédé selon la revendication 5, dans lequel l'eau contenant des bulles ultrafines est telle que la concentration de particules de bulles ayant un diamètre de particule inférieur ou égal à 1 000 nm est d'au moins $5 \times 10^7$ bulles par millilitre.

7.  Procédé selon l'une des revendications 5 et 6, dans lequel les bulles ultrafines contiennent un ou plusieurs gaz choisi(s) parmi l'air, l'oxygène, l'hydrogène, l'azote, le dioxyde de carbone, l'argon, le néon, le xénon, des gaz fluorés et des gaz inertes.

8.  Procédé selon la revendication 5 ou la revendication 7, dans lequel l'enzyme est la peroxydase, la protéase, la cellulose, l'amylase ou la lipase.

Fig.1

Fig.2

[FIG. 3]

Catalase stability (after 15-min reaction)

[FIG. 4]

Lipase stability test

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI670798 A
- JP HEI8187095 A
- JP 2011067202 A
- JP 2009225748 A
- JP 2003071472 A
- WO 2008081948 A
- JP 2008049243 A
- JP 2007105677 A
- JP 4118939 B

### Non-patent literature cited in the description

- *New Food Industry,* 1994, vol. 36 (8), 71-87
- **JOACHIM DZUBIELLA.** Explicit and implicit modeling of nanobubbles in hydrophobic confinement. *Annals of the Brazilian Academy of Sciences,* 01 March 2010, 3-12